# FASCICULE DE BREVET EUROPEEN

(11) **EP 0 718 000 B1**
(45) Date de publication et mention de la délivrance du brevet: **15.09.1999**
(21) Numéro de dépôt: 95402904.7
(22) Date de dépôt: 21.12.1995
(51) Int. Cl.: A61L 2/06, A47J 36/20

(54) **Appareil de stérilisation ou de cuisson**
Sterilisier- oder Gargerät
Sterilising or cooking apparatus

(30) Priorité: 23.12.1994 FR 9415609
(43) Date de publication de la demande: 26.06.1996
(73) Titulaire: SEB S.A., 69130 Ecully (FR)
(72) Inventeur: Doumeng, Lionel, F-74150 Marcellaz (FR)
(74) Mandataire: Keib, Gérard

(56) Documents cités:
- DE-A- 3 600 369
- DE-C- 830 385
- DE-U- 8 800 140
- FR-A- 2 324 266
- FR-A- 2 517 968
- US-A- 3 222 499
- US-A- 4 649 811

## Description

L'invention concerne un appareil de stérilisation de biberons et de cuisson d'aliments à la vapeur.

Le marché des stérilisateurs à biberons est représenté par des appareils monofonction, c'est-à-dire qui ne remplissent qu'une fonction de stérilisation, voire à double fonction, c'est-à-dire qui remplissent une seconde fonction de chauffe-biberons en plus de la fonction de stérilisation.

C'est ainsi que le document FR-A-2 583 248 décrit un chauffe-biberons stérilisateur.

On connaît également par le document DE-A- 36 00 369 un appareil permettant à la fois la stérilisation des biberons et la cuisson des oeufs. Cependant, le fonctionnement en mode cuisson vapeur est limité à la cuisson des oeufs par la forme des support plans comportant des ouvertures spécialement conçues pour supporter les oeufs.

Le but de la présente invention est de proposer un appareil présentant plusieurs modes de fonctionnement et notamment un mode de fonctionnement stérilisation et un mode de fonctionnement cuisson vapeur.

Plus particulièrement, la présente invention propose un appareil avec lequel la vapeur générée par l'ébullition de l'eau permet, dans un premier temps, de stériliser des ustensiles, et notamment les éléments constitutifs d'un biberon et dans un second temps de cuire les aliments nécessaires au repas de nourrissons, tels que légumes, poissons, riz, etc.

Plus particulièrement, la présente invention propose un appareil comprenant un minimum de pièces permettant d'assurer les fonctions de stérilisation et de cuisson vapeur recherchées, chacune des pièces ayant la forme optimale lui permettant d'assurer une double fonction.

A cet effet, l'invention concerne un appareil de stérilisation de biberons et de cuisson d'aliments à la vapeur tel que revendiqué.

D'autres caractéristiques et avantages de la présente invention apparaîtront clairement à la lecture de la description qui suit en référence aux dessins annexés dans lesquels :
- la figure 1 est une vue en coupe de la forme générale de l'appareil conforme à la présente invention ;
- la figure 2 est une vue en coupe de l'appareil conforme à la présente invention, dans son mode de fonctionnement stérilisation; et
- la figure 3 est une vue en coupe de l'appareil conforme à la présente invention, dans son mode de fonctionnement cuisson vapeur.

L'appareil conforme à la présente invention est destiné à la stérilisation des biberons et plus généralement d'ustensiles, ainsi qu'à la cuisson d'aliments notamment pour nourrissons et enfants, tels que légumes, poissons, riz...

Ces deux fonctions ont pour paramètre commun de nécessiter de la vapeur obtenue par ébullition d'eau.

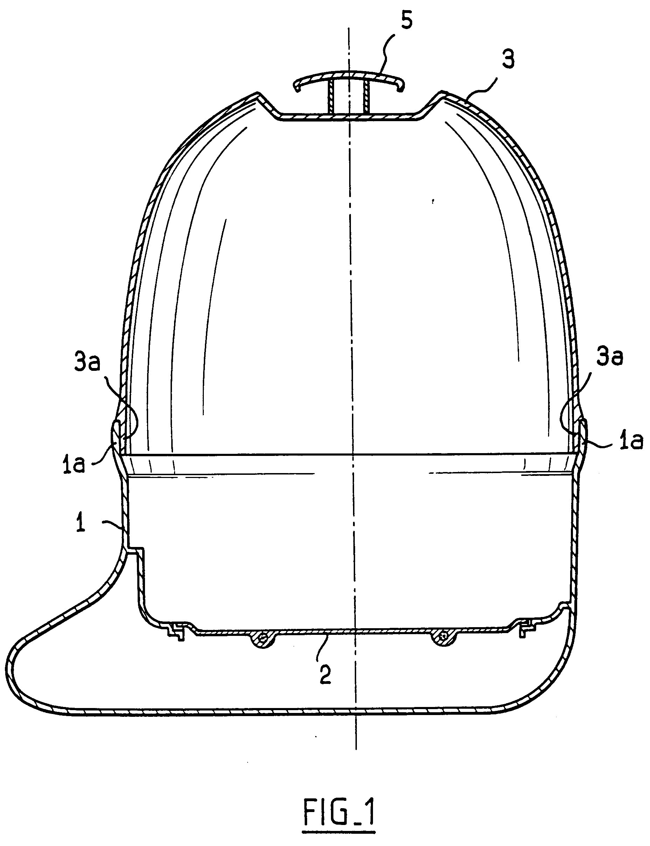

L'appareil tel que représenté à la figure 1 comporte une cuve 1 munie à son fond d'un dispositif de chauffage électrique 2 permettant de faire bouillir de l'eau et de générer de la vapeur. L'appareil comporte également un couvercle 3 équipé d'une soupape d'échappement et muni à sa partie supérieure d'une poignée 5.

L'ensemble couvercle 3 et cuve 1 définit l'enveloppe extérieure de l'appareil, le couvercle 3 étant emboîté sur la cuve 1.

La base 3a du couvercle 3 vient s'emboîter avec du jeu dans l'extrémité libre 1a évasée de la cuve 1.

Selon l'invention, l'appareil comporte des moyens 7, 10, 11 pour la stérilisation de biberons et la cuisson d'aliments à la vapeur, les moyens 7, 10, 11, permettant à l'appareil de fonctionner en mode stérilisation, et les moyens 7, 11 permettant à l'appareil de fonctionner en mode cuisson à la vapeur. Le moyen 7 est un support reposant à l'intérieur de la cuve 1. Il est destiné à supporter les éléments 14, 15, 16 constitutifs du biberon lorsque l'appareil est en mode stérilisation.

Le moyen 10 est un moyen de centrage des biberons 14 et de support des capuchons 15 et / ou des tétines 16 et / ou de tout autre ustensile à stériliser lorsque l'appareil est en mode de stérilisation. Quant au moyen 11, il se présente sous la forme d'un panier destiné en position renversée, à recevoir les tétines 16 lorsque l'appareil est en mode stérilisation, le panier étant en position normale et destiné à recevoir les aliments lorsque l'appareil est en mode cuisson vapeur.

Ainsi, lorsque l'appareil fonctionne en mode stérilisation, le support 7, le moyen de centrage 10 et le panier 11 sont utilisés ; lorsqu' il fonctionne ne mode cuisson à la vapeur, seuls le support 7 et le panier 11 sont utilisés.

Selon l'invention, le fond du panier 11 présente des perforations permettant de laisser passer la vapeur d'eau pour la stérilisation des tétines 16 lorsque l'appareil est en mode stérilisation et pour la cuisson des aliments lorsque l'appareil est en mode cuisson vapeur. En position normale, la paroi latérale 12 du panier 11 s'étend vers le fond de la cuve 1, qui se trouve sous le fond perforé du panier 11.

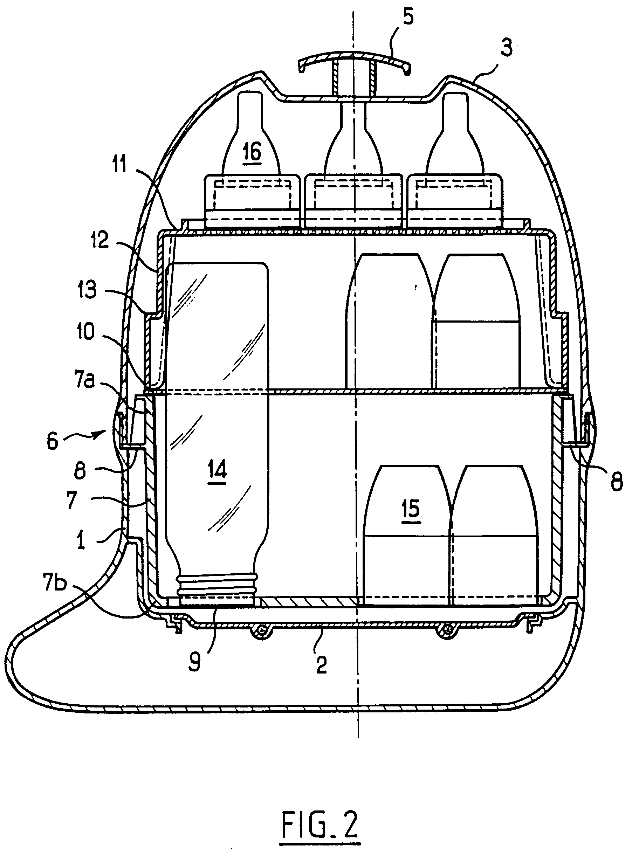

Comme illustré à la figure 2 le couvercle 3 est fixé à la cuve 1 par des moyens de fixation rapide 6. Ces derniers sont constitués par une saillie 8, notamment en forme de L inversé, saillie située vers la partie supérieure 7a du support 7 s'insérant entre l'extrémité libre 1a évasée de la cuve 1 et la base 3a du couvercle 3 lors de la fermeture de ce dernier.

Ainsi, en mode de stérilisation, illustré à la figure 2, l'appareil comprend le support 7 qui reçoit au moins un biberon 14 et des capuchons 15 et /ou tétines 16, ce support 7 maintient le moyen de centrage et de support 10 qui centre le ou les biberons 14 et peut supporter également d'autres capuchons 15. Enfin, le support 7 permet également de maintenir le panier 11 dans sa position renversée, permettant ainsi d'offrir une dernière surface susceptible de recevoir notamment des tétines 16 ou éventuellement tout autre ustensile à stériliser.

De tout ce qui précède, il ressort que l'appareil conforme à l'invention est constitué d'éléments ou de pièces dont la forme permet à chacune de remplir plusieurs fonctions, diminuant ainsi le nombre de pièces nécessaires au fonctionnement de l'appareil dans ses deux modes stérilisation et cuisson vapeur.

Pour faciliter le placement des ustensiles à stériliser, le support 7 comporte à sa partie inférieure 7b des empreintes 9 destinées à recevoir les éléments constitutifs 14, 15, 16 des biberons en mode stérilisation.

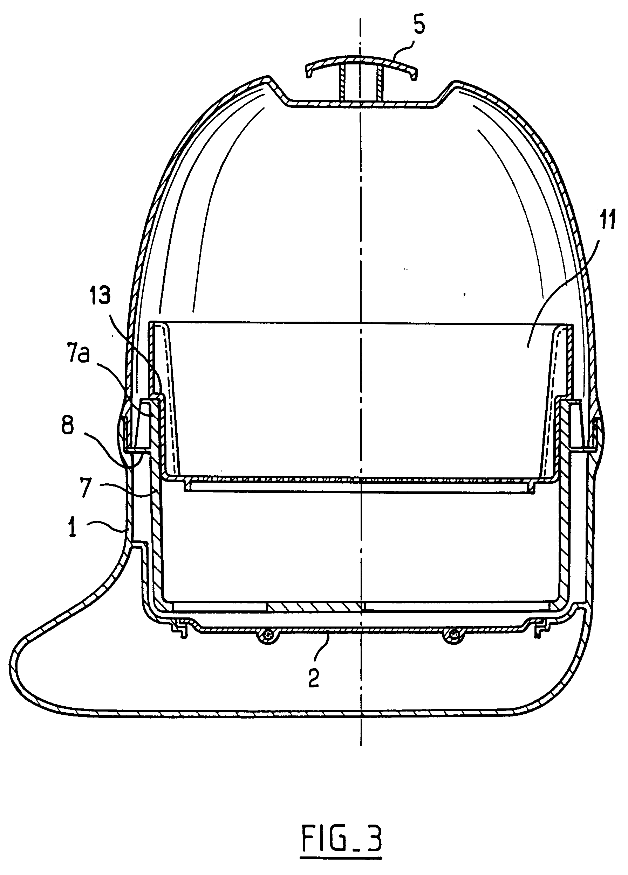

En mode cuisson vapeur, illustré à la figure 3, seuls sont utilisés le support 7 et le panier 11. Le support 7 a alors pour fonction de maintenir le panier 11 qui, dans ce mode de fonctionnement, est en position normale d'utilisation permettant ainsi de devenir un récipient destiné à recevoir les aliments à cuire.

En mode cuisson vapeur, l'appareil peut également comprendre, comme accessoire, un récipient de collecte des jus de cuisson, disposé sur le support 7 et sous le panier 11.

De manière à rapprocher le panier 11 du dispositif de chauffage électrique 2, le panier repose sur le support 7 par un décrochement 13 de sa paroi latérale 12.

On comprend de tout ce qui précède, qu'après avoir fait fonctionner l'appareil dans son mode stérilisation, l'utilisateur n'a plus qu'à enlever les ustensiles stérilisés de l'appareil ainsi que le moyen de centrage et de support 10 et le panier 11. Ensuite, l'utilisateur installe dans l'appareil, le panier 11 dans sa position normale d'utilisation. Puis, on place les aliments à cuire dans ledit panier 11. L'appareil est alors prêt à fonctionner dans son mode cuisson vapeur.

## Revendications

1. Appareil de stérilisation de biberons et de cuisson d'aliments à la vapeur comportant une cuve (1) munie son fond d'un dispositif de chauffage électrique (2), un couvercle (3) et dans l'espace défini par la cuve (1) et le couvercle (3), des moyens (7, 10, 11) pour la stérilisation de biberons et la cuisson d'aliments à la vapeur, caractérisé en ce que lesdits moyens comprennent un support (7) reposant à l'intérieur de la cuve et destiné à supporter des éléments (14, 15, 16) constitutifs de biberons lorsque l'appareil est en mode stérilisation, et un panier (11) pouvant occuper deux positions possibles, à savoir, une position renversée pour recevoir des ustensiles à stériliser, notamment des tétines (16), lorsque l'appareil est en mode stérilisation et une position normale pour recevoir des aliments lorsque l'appareil est en mode cuisson vapeur, le fond du panier (11) présentant des perforations permettant de laisser passer la vapeur d'eau.

2. Appareil confirme à la revendication 1, caractérisé en ce que lesdits moyens pour la stérilisation comprennent en outre un moyen de centrage (10) des biberons (14) et de support de capuchons (15) et / ou de tétines (16) et / ou de tout autre ustensile à stériliser lorsque l'appareil est en mode stérilisation.

3. Appareil conforme à l'une des revendications 1 ou 2, caractérisé en ce que le couvercle (3) est emboîté sur la cuve (1).

4. Appareil conforme à la revendication 3 caractérisé en ce que des moyens de fixation rapide (6) sont constitués par une saillie (8) située vers la partie supérieure (7a) du support (7) et s'insérant entre l'extrémité libre (1a) évasée de la cuve (1) et la base (3a) du couvercle (3) lors de la fermeture de ce dernier.

5. Appareil conforme à l'une quelconque des revendications 1 à 4, caractérisé en ce qu'en mode stérilisation, le support (7) maintient le moyen de centrage et de support (10) et le panier (11) en position renversée.

6. Appareil conforme à l'une quelconque des revendications 1 à 4, caractérisé en ce qu'en mode cuisson vapeur, le support (7) maintient le panier (11) en position normale d'utilisation.

7. Appareil conforme à la revendication 6, caractérisé en ce que en mode cuisson, le panier (11) repose sur le support (7) par un décrochement (13) de sa paroi latérale (12).

8. Appareil conforme à l'une des revendications 6 ou 7, caractérisé en ce qu'il comprend en outre un récipient de collecte des jus de cuisson, disposé sous le panier (11) et porté par le support (7).

9. Appareil conforme à l'une quelconque des revendications 1 à 8, caractérisé en ce que le support (7) comporte à sa partie inférieure (7b) des empreintes (9) destinées à recevoir les éléments constitutifs (14, 15, 16) des biberons en mode stérilisation.

## Claims

1. Apparatus for sterilizing babies' feeding bottles and for steaming foodstuffs, comprising a tub (1), which is fitted at the bottom with an electric heating device (2), a cover (3) and, in the space defined by the tub (1) and the cover (3), means (7, 10, 11) for sterilizing babies' feeding bottles and steaming foodstuffs, characterized in that said means comprise a support (7) resting inside the tub and designed to support the constituent parts (14, 15, 16) of the babies' feeding bottles when the apparatus is in sterilization mode, and a basket (11) which can adopt two possible positions, i.e. an upside down position for receiving items to be sterilized, more particularly teats (16), when the apparatus is in sterilization mode and a normal position for receiving foodstuffs when the apparatus is in steam-cooking mode, the bottom of the basket (11) having perforations allowing the steam to pass through.

2. Apparatus according to Claim 1, characterized in that said sterilization means also comprise a means (10) for centring the feeding bottles (14) and for supporting the tops (15) and/or teats (16) and/or any other utensil to be sterilized when the apparatus is in sterilization mode.

3. Apparatus according to one of Claims 1 or 2, characterized in that the cover (3) is fitted on to the tub (1).

4. Apparatus according to Claim 3, characterized in that quick fastening means (6) are made up of a projection (8) located towards the upper portion (7a) of the support (7) and being inserted between the free widened end (1a) of the tub (1) and the bottom (3a) of the cover (3) when closing the latter.

5. Apparatus according to any one of Claims 1 to 4, characterized in that, in sterilization mode, the support (7) holds the centring and supporting means (10) and the basket (11) in upside down position.

6. Apparatus according to any one of Claims 1 to 4, characterized in that, in steam-cooking mode, the support (7) holds the basket (11) in the normal position for use.

7. Apparatus according to Claim 6, characterized in that, in cooking mode, the basket (11) rests on the support (7) by a recessing (13) of its side wall (12).

8. Apparatus according to one of Claims 6 or 7, characterized in that it also includes a container for collecting the juices produced during cooking, this container being disposed beneath the basket (11) and carried by the support (7).

9. Apparatus according to any one of Claims 1 to 8, characterized in that, in its lower portion (7b), the support (7) has impressions (9) which are designed to receive the constituent parts (14, 15, 16) of the feeding bottles in sterilization mode.

## Patentansprüche

1. Gerät zum Sterilisieren von Säuglingsflaschen und Dampfgaren von Nahrungsmitteln mit einem Behälter (1), welcher an seinem Boden mit einer elektrischen Heizvorrichtung (2) ausgestattet ist, einem Deckel (3) und Vorrichtungen (7, 10, 11) zum Sterilisieren von Säuglingsflaschen und Dampfgaren von Nahrungsmitteln in dem von dem Behälter (1) und Deckel (3) definierten Raum, dadurch gekennzeichnet, daß die Vorrichtungen einen Träger (7), welcher im Inneren des Behälters ruht und dafür bestimmt ist, wesentliche Bestandteile (14, 15, 16) von Säuglingsflaschen zu tragen, wenn sich das Gerät im Sterilisiermodus befindet, und einen Korb (11) aufweisen, welcher zwei mögliche Positionen einnehmen kann, nämlich eine umgekehrte Position, um zu sterilisierende Gerätschaften, insbesondere Schnuller, aufzunehmen, wenn sich das Gerät im Sterilisiermodus befindet, und eine normale Position, um Nahrungsmittel aufzunehmen, wenn sich das Gerät im Dampfgarmodus befindet, wobei der Boden des Korbes (11) Durchbohrungen aufweist, welche ermöglichen, den Wasserdampf durchzulassen.

2. Gerät gemäß Anspruch 1, dadurch gekennzeichnet, daß die Vorrichtungen zum Sterilisieren außerdem eine Vorrichtung (10) zum Zentrieren der Säuglingsflaschen (14) und zum Tragen von Verschlußkappen (15) und/oder Schnullern (16) und/oder jeder anderen zu sterilisierenden Gerätschaft aufweisen, wenn sich das Gerät im Sterilisiermodus befindet.

3. Gerät gemäß einem der Ansprüche 1 oder 2, dadurch gekennzeichnet, daß der Deckel (3) auf den Behälter (1) aufgesetzt wird.

4. Gerät gemäß Anspruch 3, dadurch gekennzeichnet, daß Schnellbefestigungsvorrichtungen (6) von einem Vorsprung (8) gebildet sind, welcher in dem oberen Bereich (7a) des Trägers (7) angeordnet ist und sich zwischen das konisch erweiterte, freie Ende (1a) des Behälters (1) und die Basis (3a) des Deckels (3) während der Schließung dieses letzteren einfügt.

5. Gerät gemäß irgendeinem der Ansprüche 1 bis 4, dadurch gekennzeichnet, daß der Träger (7) im Sterilisiermodus die Zentrier- und Tragevorrichtung (10) und den Korb (11) in umgekehrter Position hält.

6. Gerät gemäß irgendeinem der Ansprüche 1 bis 4, dadurch gekennzeichnet, daß der Träger (7) im Dampfgramodus den Korb (11) in normaler Benutzungsposition hält.

7. Gerät gemäß Anspruch 6, dadurch gekennzeichnet, daß der Korb (11) im Garmodus durch einen Absatz (13) in seiner Seitenwand (12) auf dem Träger (7) ruht.

8. Gerät gemäß einem der Ansprüche 6 oder 7, dadurch gekennzeichnet, daß es außerdem einen Sammelbehälter für die Kochsäfte aufweist, welcher unter dem Korb (11) angeordnet ist und von dem Träger (7) getragen wird.

9. Gerät gemäß irgendeinem der Ansprüche 1 bis 8, dadurch gekennzeichnet, daß der Träger (7) in seinem unteren Bereich (7b) Vertiefungen (9) aufweist, welche dafür bestimmt sind, die wesentlichen Bestandteile (14, 15, 16) der Säuglingsflaschen im Sterilisiermodus aufzunehmen.
